# EUROPEAN PATENT SPECIFICATION

(11) **EP 3 057 440 B1**
(45) Date of publication and mention of the grant of the patent: **28.11.2018**
(21) Application number: 14853402.7
(22) Date of filing: 06.10.2014
(51) Int. Cl.: A23K 50/75, A23K 10/40, A23P 10/30, A61K 31/22, A61P 31/04, A23K 20/158

(54) **FORMULATION PREVENTING NECROTIC ENTERITIS IN GALLOANSERANS**
FORMULIERUNG ZUR VERHINDERUNG NEKROTISCHER ENTERITIS BEI GALLOANSERANEN
FORMULATION PRÉVENANT L'ENTÉRITE NÉCROTIQUE CHEZ DES GALLOANSERAE

(30) Priority: 14.10.2013 SE 1300647
(43) Date of publication of application: 24.08.2016
(73) Proprietor: Perstorp AB, 284 80 Perstorp (SE)
(72) Inventor: VAN IMMERSEEL, Filip, 9810 Eke (BE); SYGALL, Richard, 5561 AA Riethoven (NL); VAN DRIESSCHE, Karolien, 9240 Zele (BE); DUCATELLE, Richard, 9790 Wortegem-Petegem (BE); SCHWARZER, Conrad Gerard, 3583 Beringen (BE)
(74) Representative: Hoffmann Eitle
(86) International application number: PCT/SE2014/000124
(87) International publication number: WO 2015/057122

(56) References cited:
- EP-A1- 2 294 924
- WO-A2-2010/106488
- CN-A- 102 204 633
- CN-A- 102 204 633
- JP-A- 2004 236 597
- US-A- 3 952 107
- J J Dibner ET AL: "Use of Organic Acids as a Model to Study the Impact of Gut Microflora on Nutrition and Metabolism 1", J. Appl. Poult. Res, 1 January 2002 (2002-01-01), pages 453-463, XP055337614, Retrieved from the Internet: URL:https://academic.oup.com/japr/article- pdf/11/4/453/2260523/japoulres11-0453.pdf
- DRAGANA STANLEY ET AL: "Changes in the caecal microflora of chickens following Clostridium perfringens challenge to induce necrotic enteritis", VETERINARY MICROBIOLOGY, vol. 159, no. 1-2, 1 September 2012 (2012-09-01), pages 155-162, XP055325860, NL ISSN: 0378-1135, DOI: 10.1016/j.vetmic.2012.03.032
- SHAWKAT A. M'SADEQ ET AL: "Towards the control of necrotic enteritis in broiler chickens with in-feed antibiotics phasing-out worldwide", ANIMAL NUTRITION, vol. 1, no. 1, 1 March 2015 (2015-03-01), pages 1-11, XP055337700, ISSN: 2405-6545, DOI: 10.1016/j.aninu.2015.02.004
- STANLEY, D. ET AL.: 'Changes in the caecal microflora of chickens following Clostridium perfringens challenge to induce necrotic enteritis'.' VETERINARY MICROBIOLOGY. vol. 159, no. 1-2, 2012, pages 155 - 162, XP055325860
- R. ROY ET AL: "Influence of a propionic acid feed additive on performance of turkey poults with experimentally induced poult enteritis and mortality syndrome", POULTRY SCIENCE, vol. 81, no. 7, 1 July 2002 (2002-07-01), pages 951-957, XP55337610, Oxford ISSN: 0032-5791, DOI: 10.1093/ps/81.7.951

## Description

### FIELD OF THE INVENTION

The present invention refers to the a specific glycerol ester composition for use in preventing and/or alleviating necrotic enteritis in the gastric tract of galloanserans, wherein the necrotic enteritis is caused by Clostridium perfringens.

The glycerol ester composition comprises at least 75% by weight of glyceryl tripropionate, below 25% by weight of glyceryl dipropionate and below 8% by weight of glyceryl monopropionate. The present invention also refers to the use of said composition for modulating the gut flora of galloanserans.

### BACKGROUND OF THE INVENTION

Necrotic enteritis is an acute infection affecting galloanserans. Galloanserans are divided into the subgroups galliformes (landfowls) like chicken, turkey, grouse and pheasant, and anseriforms (waterfowls) like ducks, goose and swan. Necrotic enteritis has become an emerging problem especially among poultry and is characterized by severe necroses of intestinal mucosa. The clinical illness is usually very short and often the only signs are a sudden increase in mortality. However, birds with depression, ruffled feathers, and diarrhea may also be seen. The gross lesions are primarily found in the small intestine (jejunum), which may be ballooned, friable, and contain a foul-smelling, brown fluid.

The causative agent is the gram-positive, obligate, anaerobic bacteria *Clostridium perfringens.* There are two primary *C. perfringens* types, A and C, associated with necrotic enteritis in galloanserans. Toxins produced by the bacteria cause damage to the small intestine, but also liver lesions, and mortality.

*C perfringens* is a nearly ubiquitous bacteria readily found in soil, dust, feces, feed, and used poultry litter. It is also a normal inhabitant of the intestines of healthy galloanserans.

Development of necrotic enteritis depends on the presence of predisposing factors, such as mucosal damage caused by coccidial pathogens and feed containing high protein levels.

Because *C. perfringens* is nearly ubiquitous, it is important to prevent coccidiosis, as well as changes in the intestinal microflora that would promote its growth. This has earlier been accomplished by routinely adding antibiotics to the feed. However, since the ban of growth promoting antibiotics in the European Union in 2006, necrotic enteritis has become an emerging disease among, for instance, poultry. There is a great need for alternative methods to counteract this disease or malfunction.

An alternative to counteracting harmful bacteria like *C. perfringens* through antibiotics is to try to balance or normalize the gut flora. The gut flora consists of a complex of microorganism species that live in the digestive tract. In this context *gut* is synonymous with *intestinal* and *flora* with *microbiota* and *microflora.* The microorganisms perform a host of useful functions, such as fermenting unused energy substrates, training the immune system, preventing growth of harmful, pathogenic bacteria, regulating the development of the gut, producing vitamins for the host and producing hormones to direct the host to store fats. However, in certain conditions, some species are thought to be capable of causing diseases or malfunction by producing infection or increasing cancer risk for the host.

The present invention discloses the use of a glycerol ester composition for preventing and/or alleviating necrotic enteritis in the gastric tract of galloanserans, like chicken and turkey. The use of said composition for modulating the gut flora of galloanserans is also disclosed. Use of the composition according to the invention balances or normalizes the gut flora of galloanserans in such a way that the growth of pathogenic species is inhibited and diseases like necrotic enteritis are prevented. One mechanism behind this effect is presently believed to be that the growth of helpful bacteria is favored and that this prevents the growth of pathogenic species by competing for nutrition and attachment sites to the epithelium of the colon.

The positive effects of butyric acid on gut health in poultry and other animals have been known for a long time. Different distribution forms of butyric acid have also been explored, among them distributing butyric acid in the form of glycerol esters. In recent years, a lot of interest has been directed to the use of glyceryl monobutyrate as a feed-additive to promote animal gut health.

DRAGANA STANLEY ET AL: "Changes in the caecal microflora of chickens following Clostridium perfringens challenge to induce necrotic enteritis", VETERINARY MICROBIOLOGY, vol. 159, no. 1-2, 1 September 2012 (2012-09-01), pages 155-162, discusses possible NE treatment with butyrates.

Use of a glycerol ester composition, comprising mainly glyceryl tripropionate, has now surprisingly been found to be a more efficient way of preventing necrotic enteritis in galloanserans, such as broiler chicken, compared to giving the animal glyceryl monobutyrate.

### DETAILED DESCRIPTION OF THE INVENTION

The present invention refers to the use of a glycerol ester composition comprising a short chain fatty acid, which has proven to be an efficient way of preventing necrotic enteritis in galloanserans, such as broiler chicken. The glycerol ester composition comprises at least 75% by weight of glyceryl tripropionate, below 25% by weight of glyceryl dipropionate and below 8% by weight of glyceryl monopropionate. The present invention shows that using the composition according to the present invention is a more efficient tool for preventing and/or alleviating necrotic enteritis in galloanserans than using the in the art well known glyceryl monobutyrate for the same purpose.

According to one embodiment of the present invention the glycerol ester composition comprises at least 80% by weight of glyceryl tripropionate, below 20% by weight of glyceryl dipropionate and below 5% by weight of glyceryl monopropionate.

According to a preferred embodiment of the present invention the glycerol ester composition comprises at least 85% by weight of glyceryl tripropionate, below 15% by weight of glyceryl dipropionate and below 4% by weight of glyceryl monopropionate.

According to a more preferred embodiment of the present invention the glycerol ester composition comprises at least 90% by weight of glyceryl tripropionate, below 10% by weight of glyceryl dipropionate and below 2.5% by weight of glyceryl monopropionate.

Free propionic acid is both corrosive and has a pungent odor, which causes handling problems. These problems can be avoided by distributing the propionic acid in the form of glycerol esters. According to one embodiment of the present invention, the amount of free propionic acid in the glycerol ester composition is below 1%, preferably below 0.5% and most preferably below 0.2% by weight. Keeping down the amount of free propionic acid also ensures that the pH in the glycerol ester composition is kept at a level where the glycerol ester will not undergo hydrolyzation into glycerol and free acid, hence, the product is kept stable.

According to one embodiment of the present invention, the glycerol ester composition is adsorbed on an inert carrier, such as a silica carrier. This allows the composition to be distributed as a dry product. Such a silica carrier preferably comprises porous silica particles with an average particle size of 20-70 µm. According to one embodiment of the present invention, the glycerol ester composition is adsorbed on silica particles in a weight ratio of 50-80 % glycerol ester and 20-50 % silica particles.

The glycerol ester composition according to the present invention can be added to any commercially available feedstuffs for galloanserans. The glycerol ester composition may be incorporated directly into commercially available feeds or fed supplementary to commercially available feeds.

According to one embodiment of the present invention the amount of glycerol ester composition fed to the galloanserans is from 0.05 to 1.2% by weight, preferably from 0.1 to 0.8% by weight and most preferably from 0.2 to 0.6% by weight of the galloanserans' daily feed ration.

According to one embodiment of the present invention the dosage level of said glycerol ester composition is started at a first high level which is incrementally decreased over a 1-5 week period to a last low level. The first high level is typically from 0.4 to 0.7% by weight and the last low level is typically from 0.1 to 0.5% by weight.

According to yet another embodiment of the present invention the dosage level is decreased at time intervals of 5-10 days and the decrease of the effective amount given (from one interval to the next) is preferably from 10 to 50 %.

### EMBODIMENT EXAMPLES

### Example 1

In vivo trial on the efficacy of glyceryl tripropionate vs glyceryl monobutyrate to prevent *Clostridium perfringens*-induced necrotic enteritis.

### Bacterial strains and vaccines

The challenge strain used in the in vivo trials, *C. perfringens* strain 56, was isolated from the gut of a broiler chicken with necrotic lesions from a flock with weight gain problems and has been shown to be highly virulent in earlier in vivo trials. The strain was classified as a type A strain (netB positive, beta-2 and enterotoxin negative) and produces moderate amounts of alpha toxin in vitro (Gholamiandehkordi et al., 2006).

For inoculation, the strain was grown for 24 h in Brain Heart Infusion broth (BHI, Oxoid, Basingstoke, England).

A ten-fold dose of the anticoccidial vaccine Paracox® - 8 (Schering-Plough Animal Health, Brussels, Belgium), containing live, attenuated oocysts of Eimeria (E.) acervulina (two lines), E. brunetti, E. maxima, E. necatrix, E. praecox, E. mitis and E. tenella was used in this study. Nobilis Gumboro D 78 vaccine (Schering-Plough Animal Health, Brussels, Belgium) was given in the drinking water.

### Animals and housing

In this experiment, 113 Ross broilers of mixed sex were used. They were obtained at 1 day old from a commercial hatchery. Before the trial, all rooms were decontaminated with Metatectyl® (Clim'oMedic) and with a commercial anticoccidial disinfectant (Bi-OO-cyst®; Biolink Ltd, York, UK). The birds were divided in 4 cages of 1,5 m², on wood shavings. They were given drinking water and feed ad libitum. A 23 h/1 h light darkness program was applied.

### Experimental study design:

The first 7 days, the chickens were fed a starter diet and from day 8 until 15, a grower diet. Both the starter and the grower diet were a wheat/rye (43%/7.5%) based diet, with soybean meal as protein source. From day 17 onwards, the same diet was used with the exception that fishmeal (30%) was used as a protein source. The feed was provided by the Institute for Agricultural and Fisheries Research (ILVO). The tested products were mixed in the feed. The Gumboro vaccine was given in the drinking water at day 16 in all groups. All groups were challenged orally one time a day with approximately 4.10⁸ cfu *C. perfringens* strain 56 at days 17, 18, 19 and 20. At day 18 all birds were orally inoculated with a ten-fold dose of Paracox-8™. At days 21, 22 and 23, 9 animals of each group were euthanized by intravenous sodium pentobarbital injection.

### Model:

**Table 1. Time schedule for Experiment 1.**

| | d16 | d17 | d18 | d19 | d20 | d21 | d22 | d23 |
|---|---|---|---|---|---|---|---|---|
| Gumboro | x | | | | | | | |
| Feed + fishmeal | | x | x | x | x | x | x | x |
| Inoculation C. perfringens | | x | x | x | x | | | |
| Paracox ® x10 | | | x | | | | | |
| Scoring | | | | | | x | x | x |

### Products tested:

- Pen 1: positive control
- Pen 2: glyceryl monobutyrate (adsorbed on a silica carrier), added to the feed at a lower concentration
- Pen 3: glyceryl monobutyrate (adsorbed on a silica carrier), added to the feed at a higher concentration
- Pen 4: glyceryl tripropionate (adsorbed on a silica carrier)

The products were added to the feed at the concentrations shown in Table 2 below:

**Table 2. Concentrations ofproducts added to the feed (kg/ton)**

| | Glyceryl monobutyrate on silica carrier, low conc | Glyceryl monobutyrate on silica carrier, high conc | Glyceryl tripropionate on silica carrier |
|---|---|---|---|
| Week 1 | 5 | 7.5 | 5 |
| Week 2 | 2.5 | 6 | 2.5 |
| Week 3 | 2 | 5.5 | 2 |

Intestinal lesions in the small intestine (duodenum to ileum) were scored blinded as follows:
0: no gross lesions
1: congested intestinal mucosa
2: small focal necrosis or ulceration (1-5 foci)
3: focal necrosis or ulceration (5-16 foci)
4: focal necrosis or ulceration (16 or more foci)
5: patches of necrosis 2-3 cm long
6: diffuse necrosis typical of field cases

Lesion scores of 2 or more were classified as necrotic enteritis positive

### Statistical analysis

The GraphPad Prism Software, Inc was used to determine whether there were significant differences between groups. Statistical significance was determined at a P value of <0.05.

### Clinical observations

No abnormal clinical observations were observed.
3 chickens died during the trial.
- 1 chicken died in pen 1
- 2 chickens died in pen 3

Table 3 shows the number of birds with necrotic enteritis lesions for each group, at day 21, day 22 and day 23. Also, the total number of birds with lesions per group is shown.

**Table 3. Number of birds with macroscopic necrotic enteritis lesions on the three sampling days.**

| | pos control | glyceryl monobutyrate, low | glyceryl monobutyrate, high | glyceryl tripropionate |
|---|---|---|---|---|
| day 1 | 9/9 | 5/9 | 4/9 | 2/9 |
| day 2 | 4/9 | 3/9 | 5/9 | 3/9 |
| day 3 | 4/10 | 2/10 | 3/8 | 1/10 |
| total | 17/28 | 10/28 | 12/26 | 6/28 |
| total (%) | 61 | 36 | 46 | 21 |

The percentage of positive chickens (with macroscopic lesion score =2) is shown in Figure 1.

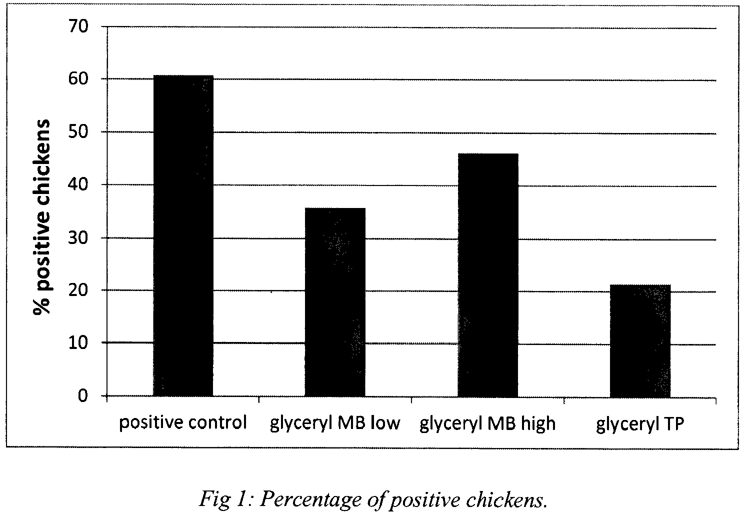

The results in Figure 1 states that adding glyceryl tripropionate to the feed gives a significantly better result, with respect to preventing *Clostridium perfringens-*induced necrotic enteritis in galloanserans, than adding glyceryl monobutyrate at either one of the two concentrations tested (21% positive chickens for glyceryl tripropionate compared to 36% positive chicken in total for glyceryl monobutyrate at the lower concentration and 46% positive chicken in total for glyceryl monobutyrate at the higher concentration).

It is also remarkable that adding a lower concentration of glyceryl monobutyrate gives a better result than adding a higher concentration (36% positive chicken in total for glyceryl monobutyrate at the lower concentration compared to 46% positive chicken in total for glyceryl monobutyrate at the higher concentration).

The results show that using a glycerol ester composition according to the present invention is an effective way of preventing *Clostridium perfringens-*induced necrotic enteritis in galloanserans.

### Example 2

In vivo trial on the efficacy of adding different concentrations of glyceryl tripropionate to the feed to control *Clostridium perfringens-*induced necrotic enteritis.

Experiment 1 was repeated, but this time different concentrations of glyceryl tripropionate was added to the animal feed. In this experiment, 169 Ross broilers of mixed sex were used and the birds were divided in 5 cages:
- Pen 1: positive control
- Pens 2-6: glyceryl tripropionate (adsorbed on a silica carrier), supplied at different concentrations

Glyceryl tripropionate was added to the feed at the concentrations shown in Table 4 below:

**Table 4. Concentrations of glyceryl tripropionate added to the feed (kg/ton)**

| Product | Glyceryl tripropionate 1 on silica carrier | Glyceryl tripropionate 2 on silica carrier | Glyceryl tripropionate 3 on silica carrier | Glyceryl tripropionate 4 on silica carrier | Glyceryl tripropionate 5 on silica carrier |
|---|---|---|---|---|---|
| Concentration (kg/ton) | | | | | |
| Week 1 | 1 | 3 | 5 | 6 | 7.5 |
| Week 2 | 0.5 | 1.5 | 2.5 | 4 | 6 |
| Week 3 | 0.35 | 1 | 2 | 3.5 | 5.5 |

### Clinical observations

No abnormal clinical observations were observed.
3 chickens died during the trial.
- 1 chicken died in pens 1, 5 and 6 each.

Table 3 shows the number of birds with necrotic enteritis lesions for each group, at day 21, day 22 and day 23. Also, the total number of birds with lesions per group is shown.

**Table 5. Number of birds with macroscopic necrotic enteritis lesions on the three sampling days.**

| | Pos control | Triprop 1 | Triprop 2 | Triprop 3 | Triprop 4 | Triprop 5 |
|---|---|---|---|---|---|---|
| Day 1 | 3/9 | 4/9 | 3/9 | 2/9 | 1/9 | 1/9 |
| Day 2 | 5/9 | 3/10 | 2/9 | 2/9 | 3/9 | 2/9 |
| Day 3 | 2/9 | 3/10 | 1/10 | 0/10 | 0/9 | 2/9 |
| Total | 10/27 | 10/29 | 6/28 | 4/28 | 4/27 | 5/27 |
| Total (%) | 37 | 34 | 21 | 14 | 15 | 19 |

The percentage of positive chickens (with macroscopic lesion score =2) is shown in Figure 2.

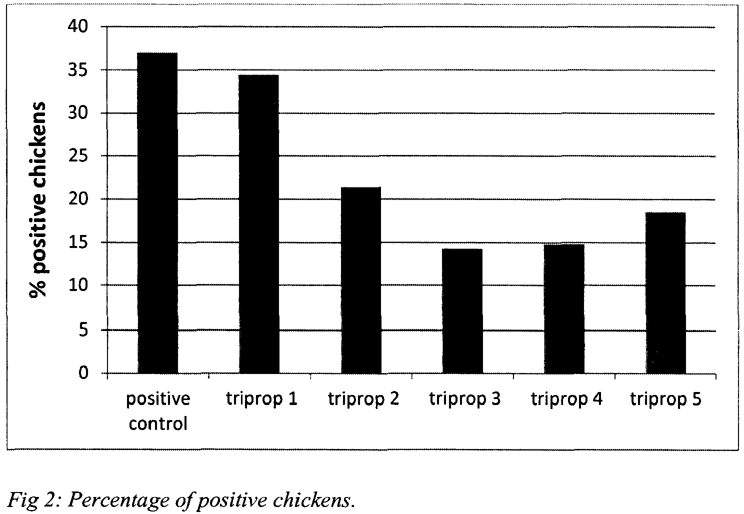

Figure 2 shows that the feeding concentrations of glyceryl tripropionate called "triprop 3" (starting at 5 kg/ton) and "triprop 4" (starting at 6 kg/ton) are the ones that are most effective for preventing necrotic enteritis in chicken (14% and 15% positive chicken for "triprop 3" and "triprop 4" respectively). These results constitute important information about how glyceryl tripropionate should be fed to galloanserans in order to prevent necrotic enteritis effectively. The results show that the optimum concentration series is somewhere around "triprop 3" and "triprop 4" and that both lower and higher concentrations than these give poorer results when it comes to preventing necrotic enteritis in galloanserans.

The glycerol ester composition may for example comprise a blend of glycerol esters of different short chain fatty acids, such as propionic acid, butyric acid and valeric acid. The dosage levels will then have to be calculated with respect to the total amount of glycerol esters.

It may further prove to be useful to feed said glycerol ester composition to the galloanserans for a shorter or longer period of time and to extrapolate the dosage levels accordingly.

## Claims

1. A glycerol ester composition comprising at least 75% by weight of glyceryl tripropionate, below 25% by weight of glyceryl dipropionate and below 8% by weight of glyceryl monopropionate, for use in prevention and/or alleviation of necrotic enteritis in the gastric tract of galloanserans, wherein the necrotic enteritis is caused by *Clostridium perfringens.*

2. A glycerol ester composition for use according to claim 1, wherein said galloanserans are galliformes, such as chicken, turkey, grouse or pheasant.

3. A glycerol ester composition for use according to claim 1, wherein said galloanserans are anseriforms, such as ducks, goose or swan.

4. A glycerol ester composition for use according to any of the claims 1-3, wherein said glycerol ester composition comprises at least 80% by weight of glyceryl tripropionate, below 20% by weight of glyceryl dipropionate and below 5% by weight of glyceryl monopropionate.

5. A glycerol ester composition for use according to any of the claims 1-3, wherein said glycerol ester composition comprises at least 85% by weight of glyceryl tripropionate, below 15% by weight of glyceryl dipropionate and below 4% by weight of glyceryl monopropionate.

6. A glycerol ester composition for use according to any of the claims 1-3, wherein said glycerol ester composition comprises at least 90% by weight of glyceryl tripropionate, below 10% by weight of glyceryl dipropionate and below 2.5% by weight of glyceryl monopropionate.

7. A glycerol ester composition for use according to any of the claims 1-6, wherein the amount of free propionic acid in said glycerol ester composition is below 1 % by weight, preferably below 0.5% by weight and most preferably below 0.2% by weight.

8. A glycerol ester composition for use according to any of the claims 1-7, wherein said glycerol ester composition is adsorbed on a carrier, such as a silica carrier, thereby allowing said glycerol ester composition to be distributed as a dry product.

9. A glycerol ester composition for use according to claim 8, wherein said carrier comprises porous silica particles with an average particle size of 20-70 µm.

10. A glycerol ester composition for use according to claim 8, wherein said glycerol ester composition is adsorbed on silica particles in a weight ratio of 50-80 % glycerol ester composition and 20-50 % silica particles.

11. A glycerol ester composition for use according to any of the claims 1-10, wherein said glycerol ester composition is fed to said galloanserans in an amount between 0.05 to 1.2 % by weight, preferably between 0.1 to 0.8 % by weight and most preferably between 0.2 to 0.6 % by weight of the galloanserans' daily feed ration.

12. A glycerol ester composition for use according to claim 11, wherein said amount of said glycerol ester composition is administered over time intervals of 5-10 days and wherein said amount is decreased over a 1-5 week period.

13. A glycerol ester composition for use according to claim 12, wherein the dosage level of said glycerol ester composition is started at a first high level, which is typically from 0.4 to 0.7% by weight, and which is incrementally decreased to a last low level, which is typically from 0.1 to 0.5% by weight.

14. A glycerol ester composition for use according to claim 12 or 13, wherein the decrease of said amount is 10-50% of the amount given during the previous interval.

## Patentansprüche

1. Glycerinesterzusammensetzung, die wenigstens 75 Gew.% Glyceryltripropionat, unter 25 Gew.% Glyceryldipropionat und unter 8 Gew.% Glycerylmonopropionat umfasst, zur Verwendung bei der Prävention und/oder Linderung von nekrotischer Enteritis im Verdauungstrakt von Galloanserae, wobei die nekrotische Enteritis durch *Clostridium perfringens* verursacht wird.

2. Glycerinesterzusammensetzung zur Verwendung gemäß Anspruch 1, wobei die Galloanserae Galliformes sind, wie etwa Huhn, Truthahn, Raufußhuhn oder Fasan.

3. Glycerinesterzusammensetzung zur Verwendung gemäß Anspruch 1, wobei die Galloanserans Anseriformes sind, wie etwa Enten, Gans oder Schwan.

4. Glycerinesterzusammensetzung zur Verwendung gemäß einem der Ansprüche 1 bis 3, wobei die Glycerinesterzusammensetzung wenigstens 80 Gew.% Glyceryltripropionat, unter 20 Gew.% Glyceryldipropionat und unter 5 Gew.% Glycerylmonopropionat umfasst.

5. Glycerinesterzusammensetzung zur Verwendung gemäß einem der Ansprüche 1 bis 3, wobei die Glycerinesterzusammensetzung wenigstens 85 Gew.% Glyceryltripropionat, unter 15 Gew.% Glyceryldipropionat und unter 4 Gew.% Glycerylmonopropionat umfasst.

6. Glycerinesterzusammensetzung zur Verwendung gemäß einem der Ansprüche 1 bis 3, wobei die Glycerinesterzusammensetzung wenigstens 90 Gew.% Glyceryltripropionat, unter 10 Gew.% Glyceryldipropionat und unter 2,5 Gew.% Glycerylmonopropionat umfasst.

7. Glycerinesterzusammensetzung zur Verwendung gemäß einem der Ansprüche 1 bis 6, wobei der Gehalt an freier Propionsäure in der Glycerinesterzusammensetzung unter 1 Gew.%, vorzugsweise unter 0,5 Gew.% und am meisten bevorzugt unter 0,2 Gew.% beträgt.

8. Glycerinesterzusammensetzung zur Verwendung gemäß einem der Ansprüche 1 bis 7, wobei die Glycerinesterzusammensetzung auf einem Träger, wie etwa einem Siliciumdioxidträger, adsorbiert ist, wodurch ermöglicht wird, dass die Glycerinesterzusammensetzung als ein Trockenprodukt verteilt werden kann.

9. Glycerinesterzusammensetzung zur Verwendung gemäß Anspruch 8, wobei der Träger poröse Siliciumdioxidpartikel mit einer durchschnittlichen Partikelgröße von 20 bis 70 µm umfasst.

10. Glycerinesterzusammensetzung zur Verwendung gemäß Anspruch 8, wobei die Glycerinesterzusammensetzung auf Siliciumdioxidpartikeln in einem Gewichtsverhältnis von 50 bis 80 % Glycerinesterzusammensetzung und 20 bis 50 % Siliciumdioxidpartikeln adsorbiert ist.

11. Glycerinesterzusammensetzung zur Verwendung gemäß einem der Ansprüche 1 bis 10, wobei die Glycerinesterzusammensetzung den genannten Galloanserae in einer Menge zwischen 0,05 bis 1,2 Gew.%, vorzugsweise zwischen 0,1 bis 0,8 Gew.% und am meisten bevorzugt zwischen 0,2 bis 0,6 Gew.% der täglichen Futterration der Galloanserae verfüttert wird.

12. Glycerinesterzusammensetzung zur Verwendung gemäß Anspruch 11, wobei die genannte Menge der Glycerinesterzusammensetzung über Zeitintervalle von 5 bis 10 Tagen verabreicht wird und wobei die genannte Menge über eine Dauer von 1 bis 5 Wochen verringert wird.

13. Glycerinesterzusammensetzung zur Verwendung gemäß Anspruch 12, wobei das Dosisniveau der Glycerinesterzusammensetzung bei einem ersten hohen Niveau gestartet wird, das typischerweise von 0,4 bis 0,7 Gew.% beträgt, und das inkrementell bis zu einem letzten tiefen Niveau verringert wird, das typischerweise von 0,1 bis 0,5 Gew.% beträgt.

14. Glycerinesterzusammensetzung zur Verwendung gemäß Anspruch 12 oder 13, wobei die Verringerung der Menge 10 bis 50 % der während des vorhergehenden Intervalls gegebenen Menge beträgt.

## Revendications

1. Composition d'ester de glycérol comprenant au moins 75% en poids de tripropionate de glycéryl, moins de 25% en poids de dipropionate de glycéryl et moins de 8% en poids de monopropionate de glycéryl, pour l'utilisation dans la prévention et/ou le soulagement de l'entérite nécrotique dans le tractus gastrique de galloanserans, dans laquelle l'entérite nécrotique est causée par *Clostridium perfringens.*

2. Composition d'ester de glycérol pour l'utilisation selon la revendication 1, dans laquelle lesdits galloanserans sont des galliformes, tels que des poulets, des dindons, des gélinottes ou des faisans.

3. Composition d'ester de glycérol pour l'utilisation selon la revendication 1, dans laquelle lesdits galloanserans sont des ansériformes, tels que des canards, des oies ou des cygnes.

4. Composition d'ester de glycérol pour l'utilisation selon l'une quelconque des revendications 1-3, dans laquelle ladite composition d'ester de glycérol comprend au moins 80% en poids de tripropionate de glycéryl, moins de 20% en poids de dipropionate de glycéryl et moins de 5% en poids de monopropionate de glycéryl.

5. Composition d'ester de glycérol pour l'utilisation selon l'une quelconque des revendications 1-3, dans laquelle ladite composition d'ester de glycérol comprend au moins 85% en poids de tripropionate de glycéryl, moins de 15% en poids de dipropionate de glycéryl et moins de 4% en poids de monopropionate de glycéryl.

6. Composition d'ester de glycérol pour l'utilisation selon l'une quelconque des revendications 1-3, dans laquelle ladite composition d'ester de glycérol comprend au moins 90% en poids de tripropionate de glycéryl, moins de 10% en poids de dipropionate de glycéryl et moins de 2,5% en poids de monopropionate de glycéryl.

7. Composition d'ester de glycérol pour l'utilisation selon l'une quelconque des revendications 1-6, dans laquelle la quantité d'acide propionique libre dans ladite composition d'ester de glycérol est inférieure à 1% en poids, préférablement inférieure à 0,5% en poids et plus préférablement inférieure à 0,2% en poids.

8. Composition d'ester de glycérol pour l'utilisation selon l'une quelconque des revendications 1-7, dans laquelle ladite composition d'ester de glycérol est adsorbée sur un support, tel qu'un support de silice, permettant ainsi à ladite composition d'ester de glycérol d'être distribuée en un produit sec.

9. Composition d'ester de glycérol pour l'utilisation selon la revendication 8, dans laquelle ledit support comprend des particules de silice poreuses avec une taille de particule moyenne de 20-70 µm.

10. Composition d'ester de glycérol pour l'utilisation selon la revendication 8, dans laquelle ladite composition d'ester de glycérol est adsorbée sur des particules de silice dans un rapport en poids de 50-80% de composition d'ester de glycérol et 20-50% de particules de silice.

11. Composition d'ester de glycérol pour l'utilisation selon l'une quelconque des revendications 1-10, dans laquelle ladite composition d'ester de glycérol est alimentée auxdits galloanserans dans une quantité entre 0,05 à 1,2 % en poids, préférablement entre 0,1 à 0,8 % en poids et plus préférablement entre 0,2 à 0,6 % en poids de la ration alimentaire quotidienne des galloanserans.

12. Composition d'ester de glycérol pour l'utilisation selon la revendication 11, dans laquelle ladite quantité de ladite composition d'ester de glycérol est administrée sur des intervalles de temps de 5-10 jours et dans laquelle ladite quantité est diminuée sur une période de 1-5 semaines.

13. Composition d'ester de glycérol pour l'utilisation selon la revendication 12, dans laquelle le niveau de dosage de ladite composition d'ester de glycérol est commencé à un premier haut niveau, qui est typiquement de 0,4 à 0,7% en poids, et qui est diminué progressivement à un dernier bas niveau, qui est typiquement de 0,1 à 0,5% en poids.

14. Composition d'ester de glycérol pour l'utilisation selon la revendication 12 ou 13, dans laquelle la diminution de ladite quantité est de 10-50% de la quantité donnée pendant l'intervalle précédent.
